# EUROPEAN PATENT SPECIFICATION

(11) **EP 2 997 379 B1**
(45) Date of publication and mention of the grant of the patent: **11.11.2020**
(21) Application number: 14725104.5
(22) Date of filing: 15.05.2014
(51) Int. Cl.: G01N 33/68

(54) **METHOD FOR IN VITRO DIAGNOSIS OF A NEUROLOGICAL DISORDER**
VERFAHREN ZUR IN-VITRO-DIAGNOSE EINER NEUROLOGISCHEN ERKRANKUNG
PROCÉDÉ DE DIAGNOSTIC IN VITRO D'UN TROUBLE NEUROLOGIQUE

(30) Priority: 15.05.2013 EP 13305614
(43) Date of publication of application: 23.03.2016
(73) Proprietor: Centre Hospitalier Universitaire de Montpellier, 34000 Montpellier (FR)
(72) Inventor: LEHMANN, Sylvain, F-34170 Castelnau Le Lez (FR); DAUVILLIERS, Yves, F-34830 Clapiers (FR); GABELLE, Audrey, F-34000 Montpellier (FR)
(74) Representative: Regimbeau
(86) International application number: PCT/EP2014/059921
(87) International publication number: WO 2014/184282

(56) References cited:
- WO-A1-2011/109503
- SCHMIDT F.M. ET AL.: "Cerebrospinal fluid melanin-concentrating hormone (MCH) and hypocretin-1 (HCRT-1, Orexin-A) in Alzheimer's disease", PLOS ONE, vol. 8, no. 5, E63136, 7 May 2013 (2013-05-07), pages 1-6, XP002713687, cited in the application
- WENNSTROEM M. ET AL.: "Altered CSF orexin and [alpha]-synuclein levels in dementia patients", J. ALZHEIMERS DIS., vol. 29, no. 1, January 2012 (2012-01), pages 125-132, XP009170737, cited in the application
- SLATS D. ET AL.: "Association between hypocretin-1 and amyloid-beta(42) cerebrospinal fluid levels in Alzheimer's disease and healthy controls", CURR. ALZHEIMER RES., vol. 9, no. 10, 1 December 2012 (2012-12-01), pages 1119-1125, XP009172825, cited in the application
- BAUMANN C.R. ET AL.: "Normal CSF hypocretin-1 (orexin A) levels in dementia with Lewy bodies associated with excessive daytime sleepiness", EUR. NEUROL., vol. 52, no. 2, 13 July 2004 (2004-07-13), pages 73-76, XP009172856, cited in the application
- YASUI K. ET AL.: "CSF orexin levels of Parkinson's disease, dementia with Lewy bodies, progressive supranuclear palsy and corticobasal degeneration", J. NEUROL. SCI., vol. 250, no. 1-2, December 2006 (2006-12) , pages 120-123, XP028050221, cited in the application
- GABELLE A. ET AL.: "CSF biomarkers: Proteomics investigations and clinical applications in neurodegenerative disorders", REVUE NEUROLOGIQUE, vol. 165, no. 3, March 2009 (2009-03), pages 213-222, XP009172863, cited in the application
- GABELLE A. ET AL.: "Impact of the 2008-2012 French Alzheimer plan on the use of cerebrospinal fluid biomarkers in Research Memory Center: The PLM study", J. ALZHEIMERS DIS., vol. 34, no. 1, 1 January 2013 (2013-01-01), pages 297-305, XP009172859, cited in the application
- DAUVILLIERS Y.A. ET AL.: "Hypocretin and brain beta-amyloid peptide interactions in cognitive disorders and narcolepsy", FRONT. AGING NEUROSCI., vol. 6, E119, 11 June 2014 (2014-06-11), pages 1-8, XP55130649,
- NOGUCHI-SHINOHARA M. ET AL.: "Serum tau protein as a marker for the diagnosis of Creutzfeldt-Jakob disease", J. NEUROL., vol. 258, no. 8, 1 March 2011 (2011-03-01) , pages 1464-1468,
- YILMAZ E. ET AL.: "Maternal and fetal serum orexin-A levels in gestationaldiabetes mellitus", J. OBSTET. GYNAECOL. RES., vol. 39, no. 1, January 2013 (2013-01), pages 139-145,

## Description

The present invention relates to a method for the in vitro diagnosis of a neurological disorder comprising the determination of the level or concentration of orexin-A (hypocretin-1), in a sample collected from a subject, said neurological disease being preferably Alzheimer's disease (AD) in its different forms (typical, atypical, prodromal) or mild cognitive impairment (MCI). The present invention also relates to the use of orexin-A (hypocretin-1) as a biomarker for the in vitro diagnosis of a neurological disorder, preferably AD or MCI. The present invention finally relates to kits for the in vitro diagnosis of a neurological disorder, preferably AD or MCI, comprising means for the detection of orexin-A.

Alzheimer's Disease is the most common cause of dementia in elderly populations. It is characterized by extracellular aggregation of neurotoxic β-amyloid peptides (Aβ) produced from amyloid precursor protein (APP), and intra-neuronal neurofibrillary tangles composed of abnormally phosphorylated tau associated with neuronal and synaptic losses in particular brain regions (Duyckaerts *et al*., 2009; Braak and Braak, 1996). Diagnosis means of AD include clinical observation, assessment of intellectual functioning, including memory testing, and advanced neuroimaging methods such as computed tomography (CT), magnetic resonance imaging (MRI), single photon emission computed tomography (SPECT) or positron emission tomography (PET). The diagnosis can be confirmed post-mortem when brain material is available and can be examined histologically (Marksteiner J. *et al*, 2008). CerebroSpinal Fluid (CSF) biomarkers are widely used for the diagnosis of AD in atypical clinical forms and for differential and early diagnosis (Blennow *et al.*, 2009; Mattsson *et al.,* 2009; Gabelle *et al*., 2011). Clinically used biological markers for diagnosing AD and differentiating it from other forms of dementia are β-amyloid peptides, Tau, and phosphorylated Tau (phospho-tau-181 or P-Tau). A reduced CSF level of Aβ₄₂ and a raised level of Tau and P-Tau are useful indicators in the diagnosis. Also, the levels of Aβ₄₂, Tau and P-Tau could be combined, like for example in the IATI (Innotest® Amyloid Tau Index), which is actually used for diagnosing AD.

However, there is still a need to increase the sensitivity and specificity of a diagnosis of Alzheimer's disease and of MCI. There is a need in particular to develop tools for diagnosis at the early stage of the affection, to increase differentiation of early stages of Alzheimer's disease and MCI from normal aging and to better discriminate AD and MCI form other dementia sharing similar clinical symptoms. Also, there is still a need to predict conversion from prodromal stages (mild cognitive impairment) to Alzheimer's disease and to predict the evolution rate of the disease.

Previous studies showed normal CSF orexin-A (hypocretin-1) values in patients with AD (Dauvilliers *et al.*, 2003), normal or low levels in patients with Dementia with Lewis Bodies (DLB) (Baumann *et al*., 2004; Wennström *et al.*, 2012), but with few CSF orexin studies that compare large groups of patients with cognitive impairment. One study reported a 40% reduction on total number of hypocretin neurons in AD (Fronczek *et al.*, 2011) but no correlation was yet reported on CSF hypocretin-1 levels and the number of hypocretin neurons in humans as surviving neurons might compensate for lost neurons by increasing hypocretin synthesis. Interactions were described in an amyloid precursor protein (APP) mice model of AD with increased levels of Aβ peptides during hypocretin infusion and decreased with hypocretin receptor antagonist (Kang *et al.*, 2009). A recent human study collecting CSF samples via lumbar catheters in six patients with AD and 6 controls also reported a correlation between hypocretin-1 and Aβ₄₂ (Slats *et al.*, 2013). However a largest CSF study failed to report any relationship between hypocretin-1 and Aβ₄₂ either in AD or in normal controls but underlined a correlation between hypocretin-1 and total Tau in female controls only (Wennström *et al.*, 2012). Schmidt et al. (2013) discloses the comparison of hypocretin-1 (HCRT-1, Orexin-A) levels in CSF of healthy subects and AD patients, and also showed a main effect of gender on the HCRT-1 levels, whereas diagnosis did not reach significance and CSF-HCRT-1 did not differ between groups of AD and of healthy subjects. Yasui et al. (2006) does not mention AD, and discloses that orexin levels in progressive supranuclear palsy (PSP) and in corticobasal degeneration (CBD) were significantly lower compared to Parkinson's disease (PD) patients, whereas the occurrence of low orexin levels was rare in both PD and dementia with Lewy bodies (DLB). Gabelle et al. (2009) and Gabelle et al. (2013) disclose the research of identifying CSF biomarkers in neurodegenerative disorders and cites tau, phosphorylated tau and amyloid β42. Thus the relationships between specific biomarkers of AD and orexin-A were not established yet.

The present invention provides a new method for the in vitro diagnosis of neurological disorders, preferably AD and MCI, based on the determination of the level or concentration of orexin-A, a specific fragment thereof or a nucleotide encoding the same, said nucleotide being preferably a mRNA molecule or a combination thereof. This determination can be performed in combination with the determination of the level of biomarkers of AD, preferably Aβ, Tau and P-Tau. The present invention discloses that the combination of the determination of the level of orexin-A and of Tau and P-Tau is superior for AD diagnosis, with a higher AUC (area under the ROC curve), to all known biomarkers used alone or in combination, including the IATI®. Combinaison with Aβ also improved significantly the AUC. The present invention also discloses the existence of a positive correlation between the levels of orexin-A and of Aβ₄₂, which is specific for AD and MCI but not for other types of dementia. Therefore, the present invention provides a new method and a marker that improves the sensibility and specificity of the biochemical diagnosis of AD and of MCI, possibly allowing an early diagnosis. It provides also the basis for therapeutic intervention and follow-up for AD. A method according to the invention improves the discriminative diagnosis of MCI and AD relatively to other neurological diseases and provides a tool for monitoring the progression of AD or MCI. The present invention may also be useful for sub-classifying populations of subjects affected by neurological disorders.

### DETAILED DESCRIPTION OF THE INVENTION

The present invention will become more fully understood from the detailed description given herein and from the accompanying drawings, which are given by way of illustration only and do not limit the intended scope of the invention. The invention is defined by the appended claims.

The present invention first relates to a method for the in vitro diagnosis of a neurological disorder, which is a dementia chosen in the group consisting of AD and MCI, said method comprising the steps of:
a) determining in a test sample from a subject the level or concentration of a compound chosen in the group consisting of orexin-A, a specific fragment thereof, an isoform thereof, a precursor thereof, a nucleic acid encoding the same and a combination thereof,
b) comparing the level or concentration of said compound in said test sample of step a) with the level or concentration of the same compound in a reference sample, wherein the reference sample is collected from subjects affected by a dementia excepting AD and MCI,
c) determining, from the comparison of step b), if said subject is affected by said neurological disorder.

The term "diagnosis" relates to determine if a subject is suffering from a particular affection. It is known that the diagnosis of a neurological disorder involves at least a clinical observation of the symptoms of said subject and possibly neuroimaging. Therefore, a method for the in vitro diagnosis of a neurological disorder, according to the present invention, may be considered as a tool within a diagnosis process.

The terms "orexin" and "hypocretin" designate the same neuropeptide, also designated as HCRT, which plays a significant role in the regulation of food intake and sleep-wakefulness, possibly by coordinating the complex behavioral and physiologic responses of these complementary homeostatic functions. Orexin (hypocretin) is a 131 amino-acid protein subject to a post-translational modification wherein specific enzymatic cleavages at paired basic residues yield the different active peptides. The terms "orexin-A" (hypocretin-1) and "orexin-B" (hypocretin-2) respectively refer to neuropeptide derived of orexin (hypocretin) from proteolytic processing. The terms "orexin-A" and "hypocretin-1" are therefore equivalent and are used indifferently in the present Application. Orexin-A and orexin-B are respectively 33 amino acids long and 28 amino acids long and share approximately 50% sequence identity. Orexin-A binds to both OX1R and OX2R with a high affinity, whereas orexin-B binds only to OX2R. Defects in HCRT are the cause of narcolepsy with cataplexy (Dauvilliers *et al*., 2007). Narcolepsy is a neurological disabling sleep disorder, characterized by excessive daytime sleepiness, sleep fragmentation, symptoms of abnormal rapid-eye-movement (REM) sleep, such as cataplexy, hypnagogic hallucinations, and sleep paralysis. Orexins are absent and/or greatly diminished in the brain and cerebrospinal fluid (CSF) of most narcoleptic patients with cataplexy (Dauvilliers *et al.*, 2007).
By "specific fragment thereof' it is intended a fragment of orexin-A which can be specifically detected by a specific ligand of orexin-A. Non limitating examples of specific fragments are: an epitope of orexin-A specifically binding to an anti-orexin-A antibody, or to a fragment, analog or derivative of an antibody; a fragment of orexin-A specifically binding to a receptor for orexin-A, preferably OX1R. By "specifically binding", "specifically binds", "recognizing" or the like, it is intended herein that, the peptide and its ligand form a complex that is relatively stable under physiological conditions. Specific binding can be characterized by an equilibrium dissociation constant of at least about 1x10⁶ M or less. Methods for determining whether two molecules bind are well known in the art and include, for example, equilibrium dialysis, surface plasmon resonance, and the like.
The terms "polypeptide", "peptide" and "protein" are used interchangeably herein to refer to a polymer of amino acid residues. By "isoform thereof' it is intended any isoform of orexin-A, and preferably the isoform orexin-B, wherein the term "isoform" designates different forms of a protein that may be produced from different genes, or from the same gene by alternative splicing. By "precursor thereof' it is intended any precursor of orexin-A, and preferably orexin. The term "orexin-A precursor", designates a pro-protein or pro-peptide which is an inactive protein that can be turned into an active form by posttranslational modification.

The term "sample" or "biological sample" means a biological material isolated from a subject. The determination of the "level" of a compound relates to the determination of the amount of a compound in a sample and preferably the determination of the quantity of said compound in said sample. The level of a compound may be expressed relatively to a reference sample, for example as a ratio or a percentage. The level may also be expressed as the intensity or localization of a signal, depending on the method used for the determination of said level. In a particular embodiment of the invention, the quantity of a compound is expressed as a concentration of said compound in a sample. Preferably, the concentration of a compound in a sample is expressed after normalization of the total concentration of related compounds in said sample, for example the level or concentration of a protein is expressed after normalization of the total concentration of proteins in the sample. The concentration of a compound in a method according to the invention may be determined by any method known by a person skilled in the art. According to this embodiment, the method of the invention comprises the determination of the concentration of orexin-A, a specific fragment thereof, an isoform thereof, a precursor thereof, a nucleic acid encoding the same and a combination thereof, and the comparison of said concentration with the respective concentration of orexin-A, a specific fragment thereof, an isoform thereof, a precursor thereof, a nucleic acid encoding the same or a combination thereof in a reference sample.

A "neurological disorder" is a disorder of the nervous system of a subject. The method according to the invention is a method for the in vitro diagnosis of a neurological disorder chosen in the group consisting of Alzheimer's disease and mild to severe cognitive impairment. In a more particular embodiment, the invention relates to a method for the in vitro diagnosis of Alzheimer's disease. In another particular embodiment, the invention relates to a method for the in vitro diagnosis of mild cognitive impairment. The term "mild cognitive impairment" designates a brain function syndrome also named incipient dementia, or isolated memory impairment. MCI involves the onset and evolution of cognitive impairments, with a predominant symptom of amnesia, which is beyond those expected, based on the age and education of the individual, but which are not significant enough to interfere with their daily activities. It is known that the subject affected by MCI tend to progress to Alzheimer's disease at a rate that can reach 10% to 15% per year.

By "concentration in a reference sample" it is meant a value for concentration obtained from at least one reference sample and preferably a mean value calculated from the statistical analysis of a large number of representative samples.

The method according to the present invention comprises the comparison of the level or the concentration of orexin-A, a specific fragment thereof, an isoform thereof, a precursor thereof, a nucleic acid fragment encoding the same or a combination thereof in a test sample with the level or concentration of the same compound in a reference sample, wherein said reference sample is collected from persons exhibiting clinical symptoms of a neurological disorder, except AD and MCI.

In another particular embodiment, said reference sample is collected from persons exhibiting similar clinical symptoms than AD or MCI.

In another particular embodiment, in a method according to the present invention, the reference sample is collected from a subject affected with clinical symptoms of neurological disorder chosen in the group consisting of: depression, dementia, vascular dementia, fronto-temporal dementia, semantic dementia and dementia with Lewy bodies, Creutzfeldt-Jahob disease, central hypersomnia, narcolepsy with cataplexy, narcolepsy without cataplexy, Kleine Levin syndrome, central pain disorder, eating disorders, multiple sclerosis, Parkinson's disease, Guillain-Barre syndrome, uveitis, myasthenia gravis, neuromyotonia, neuropathic diabetes, schizophrenia and other neuro-inflammatory disorders.

According a particular embodiment, the present invention relates to an in vitro diagnosis method of a neurological disease which is a dementia chosen in the group consisting of AD and MCI, comprising the determination of the level or concentration of orexin-A, a specific fragment thereof, an isoform thereof, a precursor thereof, a nucleic acid encoding the same or a combination thereof in a test sample from a subject, wherein said subject exhibits clinical symptons of cognitive impairment or dementia, except AD and MCI.

The present invention relates to a method for the in vitro diagnosis of a neurological disorder, which is a dementia chosen in the group consisting of AD and MCI, said method comprising the steps of:
a) determining in a test sample from a subject the level or concentration of a compound chosen in the group consisting of orexin-A, a specific fragment thereof, an isoform thereof, a precursor thereof, a nucleic acid encoding the same and a combination thereof,
b) comparing the level or concentration of said compound in said test sample of step a) with the level or concentration of the same compound in a reference sample, wherein the reference sample is collected from subjects affected by a dementia excepting AD and MCI,
c) determining, from the comparison of step b), if said subject is affected by said neurological disorder, wherein a higher orexin-A level is indicative of said neurological disorder.

In a more particular embodiment, the present invention relates to a method for the in vitro diagnosis of AD according to the invention, wherein a higher orexin-A level is indicative of AD.

In another more particular embodiment, the present invention relates to a method for the in vitro diagnosis of MCI according to the invention, wherein a higher orexin-A level is indicative of MCI.

In another particular embodiment, a method according to the invention comprising the detection of orexin-A, a specific fragment thereof, an isoform thereof, a precursor thereof, a nucleic acid encoding the same or a combination thereof is a part of a method for the in vitro determination of the probability of a subject affected with MCI to be affected later by AD.

In a particular embodiment, the invention relates to a method for the in vitro diagnosis of a neurological disorder, which is a dementia chosen in the group consisting of AD and MCI, said method comprising the determination of the level or concentration of orexin-A, a specific fragment thereof, an isoform thereof, a precursor thereof, a nucleic acid encoding the same or a combination thereof, and of the level or concentration of at least one compound known as a biomarker of said neurological disorder. By "biomarker" or "marker" of a disorder or a disease it is intended to designate a biochemical compound for which the presence, level, concentration or characterization is indicative of said disorder or disease. Markers in medical imaging are included in this definition, such as [N-methyl-11C]2-(4'-methylaminophenyl)-6-hydroxybenzothiazole (PiB) in PET scan.

In a particular embodiment, the invention comprises the determination of the level or concentration of orexin-A, a specific fragment thereof, an isoform thereof, a precursor thereof, a nucleic acid encoding the same or a combination thereof, and of the level or concentration of at least one biomarker of AD.

In a more particular embodiment, a biomarker of AD is chosen in the group consisting of: Tau, P-Tau, Aβ42, APP fragments, apolipoproteins (A, B, E), ADAMs, PrP, Transthyretin, PEDF, Prostaglandin-H2 D-isomerase, APLPs, Osteopontin, TDL-43, Serotransferrin, Alpha-1-antitrypsin, Retinol-binding protein, Kallikrein, Tetranectin, BACEs, cystatin, chromogranin A, chromogranin B, clusterin, VEGF, secretogranin.

In a particular embodiment, the present invention relates to a method for the in vitro diagnosis of a neurological disease, which is a dementia chosen in the group consisting of AD and MCI, said method comprising the determination of the level or concentration of orexin-A, a specific fragment thereof, an isoform thereof, a precursor thereof, a nucleic acid encoding the same or a combination thereof, and the determination of the level or concentration of at least one compound chosen in the group consisting of: Tau, P-Tau, Aβ42, a specific fragment thereof, an isoform thereof, a precursor thereof, a nucleic acid encoding the same or a combination thereof. A nucleic acid encoding Tau, P-Tau, Aβ42, or a fragment thereof is preferably a mRNA or a cDNA molecule and is defined by its nucleotide sequence.

In a more particular embodiment, the present invention relates to a method for the in vitro diagnosis of AD comprising the determination of the level or concentration of orexin-A, a specific fragment thereof, an isoform thereof, a precursor thereof, a nucleic acid encoding the same or a combination thereof, and the determination of the level or concentration of at least one compound chosen in the group consisting of: Tau, P-Tau, Aβ, a specific fragment thereof, an isoform thereof, a precursor thereof, a nucleic acid encoding the same or a combination thereof.

Tau proteins ("Tau") are the product of alternative splicing from a single gene designated as MAPT and are also named neurofibrilling tangle protein or paired helical filament Tau (UniProt ID: P10636, amino acid sequence: SEQ ID N°3, nucleotide sequence SEQ ID N°7). Tau promotes microtubule assembly and stability, and might be involved in the establishment and maintenance of neuronal polarity. The short isoforms allow plasticity of the cytoskeleton whereas the longer isoforms may preferentially play a role in its stabilization.

Tau is phosphorylated at serine and threonine residues in S-P or T-P motifs by proline-directed protein kinases (PDPK1: CDK1, CDK5, GSK3, MAPK) and at serine residues in K-X-G-S motifs by MAP/microtubule affinity-regulating kinase (MARK1 or MARK2), causing detachment from microtubules, and their disassembly. Phosphorylation normally decreases with age. In Alzheimer disease, the neuronal cytoskeleton in the brain is progressively disrupted and replaced by tangles of paired helical filaments (PHF) and straight filaments, mainly composed of hyperphosphorylated forms of Tau (PHF-Tau or AD P-Tau). O-GlcNAcylation is greatly reduced in Alzheimer disease brain cerebral cortex leading to an increase in Tau/MAPT phosphorylations.

Phosphorylated protein Tau is also designed as "Phospho-Tau" or "P-Tau". By "determination of the concentration of phosphorylated protein Tau" it is intended to designate the determination of the concentration of phosphorylated protein Tau and especially protein Tau phosphorylated on Thr-181. "Thr-181" corresponds to the most commonly admitted designation of the position of said Thr amino acid.

### Table 1 summarizes the characteristics and references of the compounds tested.

**Table 1**

| **Name** | **Description** | **Protein reference** | **Nucleic acid reference** |
|---|---|---|---|
| Orexin (HCRT) | Homo sapiens orexin, 131 amino acids | UniProt ID: P043612, SEQ ID N°1 | GeneID: 3060. Pre-pro-orexin mRNA, GenBank: AF041240 SEQ ID N°6 |
| Orexin-A | (= Hypocretin-1), aa 34 to 66 of aa sequence of Orexin | SEQ ID N°2 | |
| Tau (MAPT) | Homo sapiens microtubule-associated protein Tau, 758 aa, is further processed, may be phosphorylated on Thr 181 | UniProt ID: P10636, Isoform PNS-Tau : P10636.1 SEQ ID N°3 | GeneID: 4137. Isoform PNS-Tau GenBank: JO3778.1 SEQ ID N°7 |
| Beta-amyloid protein (APP) | Homo sapiens amyloid beta-A4 protein, at least 11 isoforms including APP770 | Isoform APP770, Uniprot ID: P05067, SEQ ID N°4 | GeneID: 351. mRNA, GenBank: Y00264.1 SEQ ID N°8 |
| Aβ42 | Beta-amyloid protein 42 (Beta-APP42), aminoacids 672 to 713 from amyloid beta-A4 protein | SEQ ID N°5 | |

Amyloid beta A4 protein (ABPP or APPI) is cleaved in at least fourteen chains including Beta-amyloid protein 42 (Beta-APP42 or Aβ42). Amyloid beta A4 protein functions as a cell surface receptor and performs physiological functions on the surface of neurons relevant to neurite growth, neuronal adhesion and axonogenesis. Beta-amyloid peptides bind to lipoproteins and apolipoproteins E and J in the CSF and to HDL particles in plasma, inhibiting metal-catalyzed oxidation of lipoproteins. Aβ42 (amino acid sequence: SEQ ID N°4, coding nucleotide sequence SEQ ID N°8, GenBank: X06989.1, UniProt ID: P05067) may activate mononuclear phagocytes in the brain and elicit inflammatory responses, and promotes both tau aggregation and TPK II-mediated phosphorylation.

In an embodiment, the present invention comprises the determination of the level or concentration of natural variants of proteins having a nucleic acid sequence at least 80%, preferably, 90%, more preferably 95% and even more preferably 98% identity with a sequence chosen in the group consisting of SEQ ID N°2. In a more particular embodiment, the present invention comprises the determination of the level or concentration of natural variants of proteins having a nucleic acid sequence at least 80%, preferably, 90%, more preferably 95% and even more preferably 98% identity with a sequence chosen in the group consisting of SEQ ID N°2 and the determination of the level or concentration of natural variants of proteins having a nucleic acid sequence at least 80%, preferably, 90%, more preferably 95% and even more preferably 98% identity with a sequence chosen in the group consisting of SEQ ID N°3 and SEQ ID N°5.

In another embodiment, the present invention comprises the determination of the level or concentration of natural variants of nucleic acids molecules having at least 80%, preferably, 90%, more preferably 95% and even more preferably 98% identity with a sequence SEQ ID N°6 and encoding for orexin-A or for a specific fragment thereof. In a more specific embodiment, the present invention comprises the determination of the level or concentration of natural variants of nucleic acids molecules having at least 80%, preferably, 90%, more preferably 95% and even more preferably 98% identity with the sequence SEQ ID N°7 and encoding for Tau or for a specific fragment thereof, and/or the determination of the level or concentration of natural variants of nucleic acids molecules having at least 80%, preferably, 90%, more preferably 95% and even more preferably 98% identity with the sequence SEQ ID N°8 and encoding for Aβ42 or for a specific fragment thereof.

As used herein the term "identity" herein means that two amino acid sequences, or nucleic acid sequences, are identical (i.e. at the amino acid by amino acid, or nucleic acid by nucleic acid basis) over the window of comparison. The term "percentage of sequence identity" is calculated by comparing two optimally aligned sequences over the window of comparison, determining the number of positions at which the identical amino acid residues occurs in both sequences to yield the number of matched positions, dividing the number of matched positions by the total number of positions in the window of comparison (i.e. the window size) and multiplying the result by 100 to yield the percentage of sequence identity. The percentage of sequence identity of an amino acid sequence can also be calculated using BLAST software with the default or user defined parameter.

In a particular embodiment, a method according to the present invention comprises the determination of: the level or concentration of orexin-A, a specific fragment thereof, an isoform thereof, a precursor thereof, a nucleic acid encoding the same or a combination thereof, and the level or concentration of Tau, a specific fragment thereof, an isoform thereof, a precursor thereof, a nucleic acid encoding the same or a combination thereof. In another particular embodiment, a method according to the present invention comprises the determination of: the level or concentration of orexin-A, a specific fragment thereof, an isoform thereof, a precursor thereof, a nucleic acid encoding the same or a combination thereof, and the level or concentration of Phospho-Tau, a specific fragment thereof, an isoform thereof, a precursor thereof, a nucleic acid encoding the same or a combination thereof, wherein Phospho-Tau is Tau phosphorylated on any of its phorphorylated amino-acid and in particular on Thr-181. In another particular embodiment, a method according to the present invention comprises the determination of: the level or concentration of orexin-A, a specific fragment thereof, an isoform thereof, a precursor thereof, a nucleic acid encoding the same or a combination thereof, and the level or concentration of Aβ42, a specific fragment thereof, an isoform thereof, a precursor thereof, a nucleic acid encoding the same or a combination thereof.

In another particular embodiment, a method according to the present invention comprises the determination of: the level or concentration of orexin-A, a specific fragment thereof, an isoform thereof, a precursor thereof, a nucleic acid encoding the same or a combination thereof, the level or concentration of Tau, a specific fragment thereof, an isoform thereof, a precursor thereof, a nucleic acid encoding the same or a combination thereof, and the level or concentration of Phospho-Tau, a specific fragment thereof, an isoform thereof, a precursor thereof, a nucleic acid encoding the same or a combination thereof. In another particular embodiment, a method according to the present invention comprises the determination of: the level or concentration of orexin-A, a specific fragment thereof, an isoform thereof, a precursor thereof, a nucleic acid encoding the same or a combination thereof, the level or concentration of Tau, a specific fragment thereof, an isoform thereof, a precursor thereof, a nucleic acid encoding the same or a combination thereof, and the level or concentration of Aβ42, a specific fragment thereof, an isoform thereof, a precursor thereof, a nucleic acid encoding the same or a combination thereof. In another particular embodiment, a method according to the present invention comprises the determination of: the level or concentration of orexin-A, a specific fragment thereof, an isoform thereof, a precursor thereof, a nucleic acid encoding the same or a combination thereof, the level or concentration of Phospho-Tau, a specific fragment thereof, an isoform thereof, a precursor thereof, a nucleic acid encoding the same or a combination thereof, and the level or concentration of Aβ42, a specific fragment thereof, an isoform thereof, a precursor thereof, a nucleic acid encoding the same or a combination thereof.

In another particular embodiment, a method according to the present invention comprises the determination of: the level or concentration of orexin-A, a specific fragment thereof, an isoform thereof, a precursor thereof, a nucleic acid encoding the same or a combination thereof, the level or concentration of Tau, a specific fragment thereof, an isoform thereof, a precursor thereof, a nucleic acid encoding the same or a combination thereof, the level or concentration of Phospho-Tau, a specific fragment thereof, an isoform thereof, a precursor thereof, a nucleic acid encoding the same or a combination thereof, and the level or concentration of Aβ42, a specific fragment thereof, an isoform thereof, a precursor thereof, a nucleic acid encoding the same or a combination thereof.

In another particular embodiment, a method according to the present invention relates to the in vitro diagnosis of a neurological disorder and comprises the determination of the concentration of: orexin-A, a specific fragment thereof, an isoform thereof, a precursor thereof, a nucleic acid encoding the same or a combination thereof, the determination of the concentration at least one biomarker for said neurological disorder, and the calculation of an index combining the concentration of orexin-A, a specific fragment thereof, an isoform thereof, a precursor thereof, a nucleic acid encoding the same or a combination thereof, and the concentration of said at least one biomarker. An index according to the invention combines the concentration of orexin-A, a specific fragment thereof, an isoform thereof, a precursor thereof, a nucleic acid encoding the same or a combination thereof, and the concentration of at least one biomarker, and is compared to a reference index value, wherein said reference index is calculated from the determination of the concentration of the same compounds in a reference sample.

In a particular embodiment, a method according to the invention comprises the calculation of an index combining the concentration of: orexin-A, a specific fragment thereof, an isoform thereof, a precursor thereof, a nucleic acid encoding the same or a combination thereof, and of Tau, a specific fragment thereof, an isoform thereof, a precursor thereof, a nucleic acid encoding the same or a combination thereof. In another particular embodiment, a method according to the invention comprises the calculation of an index combining the concentration of: orexin-A, a specific fragment thereof, an isoform thereof, a precursor thereof, a nucleic acid encoding the same or a combination thereof and of Phospho-Tau, a specific fragment thereof, an isoform thereof, a precursor thereof, a nucleic acid encoding the same or a combination thereof. In another particular embodiment, a method according to the invention comprises the calculation of an index combining the concentration of: orexin-A, a specific fragment thereof, an isoform thereof, a precursor thereof, a nucleic acid encoding the same or a combination thereof and of Aβ42, a specific fragment thereof, an isoform thereof, a precursor thereof, a nucleic acid encoding the same or a combination thereof.

In another particular embodiment, a method according to the invention comprises the calculation of an index combining the concentration: of orexin-A, a specific fragment thereof, an isoform thereof, a precursor thereof, a nucleic acid encoding the same or a combination thereof; of Tau, a specific fragment thereof, an isoform thereof, a precursor thereof, a nucleic acid encoding the same or a combination thereof and of Phospho-Tau, a specific fragment thereof, an isoform thereof, a precursor thereof, a nucleic acid encoding the same or a combination thereof. In another particular embodiment, a method according to the invention comprises the calculation of an index combining the concentration: orexin-A, a specific fragment thereof, an isoform thereof, a precursor thereof, a nucleic acid encoding the same or a combination thereof, of Tau, a specific fragment thereof, an isoform thereof, a precursor thereof, a nucleic acid encoding the same or a combination thereof and of Aβ42, a specific fragment thereof, an isoform thereof, a precursor thereof, a nucleic acid encoding the same or a combination thereof. In another particular embodiment, a method according to the invention comprises the calculation of an index combining the concentration: of orexin-A, a specific fragment thereof, an isoform thereof, a precursor thereof, a nucleic acid encoding the same or a combination thereof, of Phospho-Tau, a specific fragment thereof, an isoform thereof, a precursor thereof, a nucleic acid encoding the same or a combination thereof and of Aβ42, a specific fragment thereof, an isoform thereof, a precursor thereof, a nucleic acid encoding the same or a combination thereof.

In another particular embodiment, a method according to the invention comprises the calculation of an index combining the concentration of: orexin-A, a specific fragment thereof, an isoform thereof, a precursor thereof, a nucleic acid encoding the same or a combination thereof, of Tau, a specific fragment thereof, an isoform thereof, a precursor thereof, a nucleic acid encoding the same or a combination thereof, of Phospho-Tau, a specific fragment thereof, an isoform thereof, a precursor thereof, a nucleic acid encoding the same or a combination thereof and of Aβ42, a specific fragment thereof, an isoform thereof, a precursor thereof, a nucleic acid encoding the same or a combination thereof.

In a particular embodiment, a method according to the invention comprises the determination of the level or concentration of orexin-A, a specific fragment thereof, an isoform thereof, a precursor thereof, a nucleic acid encoding the same or a combination thereof in a test sample, wherein said test sample is a biological fluid chosen in the group consisting of: plasma, serum, whole blood, whole blood extract, and cerebrospinal fluid.

In a more particular embodiment of the invention comprises, the test sample which is cerebrospinal fluid (CSF). In an even more particular embodiment of the invention, the test sample is cerebrospinal fluid which has been collected at a precise part of the day. In an even more particular embodiment of the invention, the test sample is cerebrospinal fluid collected between 11 am and 1 pm, local time of the collection. Cerebrospinal fluid is classically collected by lumbar puncture, also called "spinal tap".

A method according to the invention comprises the preparation of biological samples and preferably comprises the protein extraction, purification and characterization, using methods well known by a person skilled in biochemical techniques. In a particular embodiment, a method of the invention comprises the extraction of orexin-A from CSF.

In a particular embodiment, the present invention comprises the determination of the level of a protein by a method chosen in the group consisting of: a method based on immuno-detection, a method based on the use of a blot, and particularly a western blot or a dot blot, a method based on chromatography, and preferably liquid chromatography, a method based on mass spectrometry and a method based on flow cytometry.

Non-limiting examples of methods based on immunodetection are immunoassays selected from the group consisting of: affinity chromatography, immunoelectrophoresis, radioimmunoassay (RIA), enzyme-linked immunoassay (ELISA), ELISA-derived assays such as immune-PCR in which the detecting antibody is labeled with a DNA-label, immunofluorescent assay, Western blotting, and the like. These methods are well known by a person skilled in the art of detecting and analyzing proteins.

In a method according to the invention, the level or concentration of a protein may be determined through direct specific binding or by indirect competitive binding to a ligand. In a particular embodiment of the invention, the determination of the concentration of orexin-A is performed via a RIA wherein orexin-A from the test sample and iodine 125-labelled orexin-A compete for binding to an anti-orexin-A antibody. Methods for the specific detection of a protein based on mass spectrometry include, but are not limited to, Selected Reaction Monitoring (SRM) and Multiple Reaction Monitoring (MRM). Methods based on flow cytometry include, but are not limited to, a multiplex assay such as Luminex®XMAP, combining flow cytometry with microspheres and lasers.

In a particular embodiment, a method according to the invention comprises the detection of the concentration of orexin-A, a specific fragment thereof, an isoform thereof, a precursor thereof, a nucleic acid encoding the same or a combination thereof and at least one compound, preferably chosen in the group consisting of Aβ42, Tau phosphorylated at threonine 181 and total Tau, or a combination thereof, wherein the concentration of said compounds is determined using using an ELISA microplate assay (INNOTEST®). In another particular embodiment, a method according to the invention comprises the detection of the concentration of orexin-A, a specific fragment thereof, an isoform thereof, a precursor thereof, a nucleic acid encoding the same or a combination thereof and at least one compound, preferably chosen in the group consisting of Aβ42, Tau phosphorylated at threonine 181 and total Tau, or a combination thereof, wherein the concentration of said compounds is determined using the multiplex xMAP Luminex platform (Luminex Corp, Austin TX) with Innogenetics immunoassay (INNOBIA AlzBio3, Ghent, Belgium)

In a particular embodiment, a method according to the invention comprises the determination of the concentration of orexin-A by performing a direct binding assay such as Surface Plasmon Resonance (SPR).

In another particular embodiment, the present invention comprises the determination of the level or the concentration of a peptide, or a specific fragment thereof, by performing a method determining the level or the concentration of a nucleic acid molecule encoding for said peptide or specific fragment of peptide in a sample, more preferably said nucleic acid molecule is a mRNA or a cDNA molecule. Methods for the specific detection of nucleic acids molecules involve methods classically used in molecular biology are well known to those skilled in the art of analyzing nucleic acids and are fully described in the literature (Maniatis T. *et al.*, Edition 1999).

In a particular embodiment, a method according to the present invention comprises the determination of the level or concentration of a nucleic acid molecule comprising a nucleotide sequence having at least 80% of identity, more preferably at least 90% identity, more preferably at least 95% of identity, even more preferably at least 99% of identity with the sequence SEQ ID N°6 and encoding for orexin-A or for a specific fragment thereof. Nucleic acid molecules comprising nucleic acid sequences having at least 80% of identity, more preferably at least 90% identity, more preferably at least 95% of identity, even more preferably at least 99% of identity with the sequence SEQ ID N°6 coding for orexin-A or for a specific fragment thereof are preferably sequences coding for the same sequences of amino acids, in relation with the degeneration of the genetic code, or complementary sequences which are capable of specifically hybridizing with sequence SEQ ID N°6 under strong stringency conditions. Strong stringency conditions means that conditions of temperature and ionic force are selected to allow the maintained hybridization between two complementary nucleic acid molecules or fragments. In an embodiment, a method according to the invention comprises the use of short oligonucleotide sequences able to specifically hybridize to a nucleic acid molecule comprising a sequence having at least 80% identity with SEQ ID N°6.

In another embodiment, the present invention relates to the use of the determination of the level or the concentration of orexin-A, a specific fragment thereof, an isoform thereof, a precursor thereof, a nucleic acid molecule encoding the same or a combination thereof in a sample as a biomarker for the in vitro diagnosis of a neurological disease. which is a dementia chosen in the group consisting of AD and MCI. In a more particular embodiment, the present invention relates to the use of the determination of the level or the concentration of orexin-A, a specific fragment thereof, an isoform thereof, a precursor thereof, a nucleic acid molecule encoding the same or a combination thereof in a sample as a biomarker for the in vitro diagnosis of MCI or for the in vitro diagnosis of AD.

In a more particular embodiment, the present invention relates to the use of the determination of the level or the concentration of orexin-A, a specific fragment thereof, an isoform thereof, a precursor thereof, a nucleic acid molecule encoding the same or a combination thereof and of the level or concentration of at least one compound chosen in the group consisting of: Tau, P-Tau, Aβ42, a specific fragment thereof, an isoform thereof, a precursor thereof, a nucleic acid molecule encoding the same or a combination thereof for the in vitro diagnosis of MCI or for the in vitro diagnosis of AD.

In another particular embodiment, the present invention relates to the use of the determination of the level or concentration of orexin-A, a specific fragment thereof, an isoform thereof, a precursor thereof, a nucleic acid molecule encoding the same or a combination thereof for the early-stage diagnosis of AD.

In a particular embodiment, a method according to the invention relates to the use of the determination of the concentration of orexin-A, a specific fragment thereof, an isoform thereof, a precursor thereof, a nucleic acid encoding the same or a combination thereof for the in vitro discriminative diagnosis of AD or MCI from normal aging symptoms.

In a particular embodiment, a method according to the invention relates to the use of the determination of the concentration of orexin-A, a specific fragment thereof, an isoform thereof, a precursor thereof, a nucleic acid encoding the same or a combination thereof for the in vitro discriminative diagnosis of AD or MCI from other neurological disorders.

In a more particular embodiment, a method according to the invention relates to the use of the determination of the concentration of orexin-A, a specific fragment thereof, an isoform thereof, a precursor thereof, a nucleic acid encoding the same or a combination thereof for the in vitro discriminative diagnosis of AD or MCI from neurological disorders chosen in the group of: dementia and depression.

In another particular embodiment, a method according to the relates to the use of the determination of the concentration of orexin-A, a specific fragment thereof, an isoform thereof, a precursor thereof, a nucleic acid encoding the same or a combination thereof for the in vitro determination of the probability of a subject affected with MCI to be affected later by AD.

In another particular embodiment, the present invention relates to the use of a kit for the in vitro diagnosis of a neurological disease chosen in the group consisting of MCI and AD, said kit comprising a reagent for the specific detection of orexin-A, a specific fragment thereof, an isoform thereof, a precursor thereof, a nucleic acid molecule encoding the same or a combination thereof, and at least one compound chosen in the group consisting of buffers, positive controls and instructions for use. A reagent for the specific detection of orexin-A may be chosen in the non-limiting list consisting of: an antibody, an antibody derivative, an antibody analog or an antibody fragment, a peptide and an aptamer specifically binding orexin-A and/or a functional fragment thereof.

In a more particular embodiment, a kit according to the invention comprises a reagent for the specific detection of orexin-A, a specific fragment thereof, an isoform thereof, a precursor thereof, a nucleic acid molecule encoding the same or a combination thereof, at least one compound chosen in the group consisting of: buffers, positive controls and instructions for use, and at least one reagent for the specific detection of a compound chosen in the group consisting of: Tau, phospho-Tau, Aβ42, orexin-A, a specific fragment thereof, an isoform thereof, a precursor thereof, a nucleic acid molecule encoding the same or a combination thereof. A reagent for the specific detection of a compound may be chosen in the non-limiting list consisting of: an antibody, an antibody derivative, an antibody analog or an antibody fragment, a peptide and an aptamer specifically binding said compound.

The following examples are provided herein for purposes of illustration only and are not intended to be limiting unless otherwise specified.

### BRIEF DESCRIPTION OF THE FIGURES

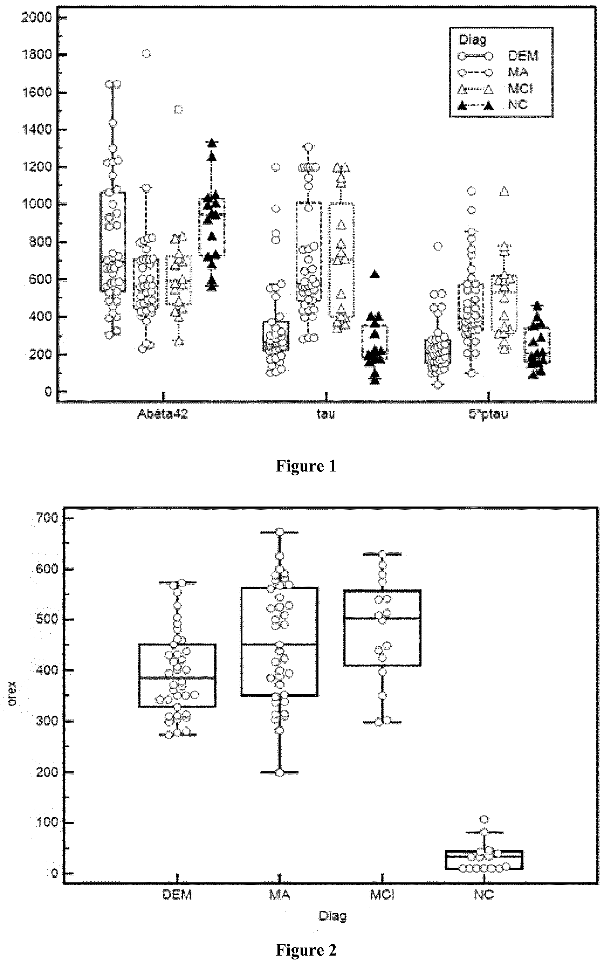

**Figure 1****:** Representation of the concentration of the biomarkers Aβ42, Tau and P-Tau in patients from various clinical populations. The concentration of biomarker is indicated on the ordinate axis in pg/ml, with Aβ42 ("Abeta42"), Tau ("tau") and P-Tau ("5*ptau") represented from left to right. For each biomarker, concentration is shown for patients suffering of Alzheimer's disease ("AD" or "MA", white circles on dotted line), amnesia mild cognitive impairment (MCI, white triangles), narcolepsy ("NC", black triangles) and other dementia ("DEM", white circles on continuous line).
**Figure 2****:** Representation of the concentration of orexin-A/hypocretin-1 in patients from various clinical populations. Orexin-A concentration is indicated on the ordinate axis in pg/ml with, from left to right, patients suffering of: other dementia ("DEM"), Alzheimer's disease ("MA"), amnesia mild cognitive impairment ("MCI") and narcolepsy ("NC"). T-test revealed significant differences between DEM and AD or MCI.

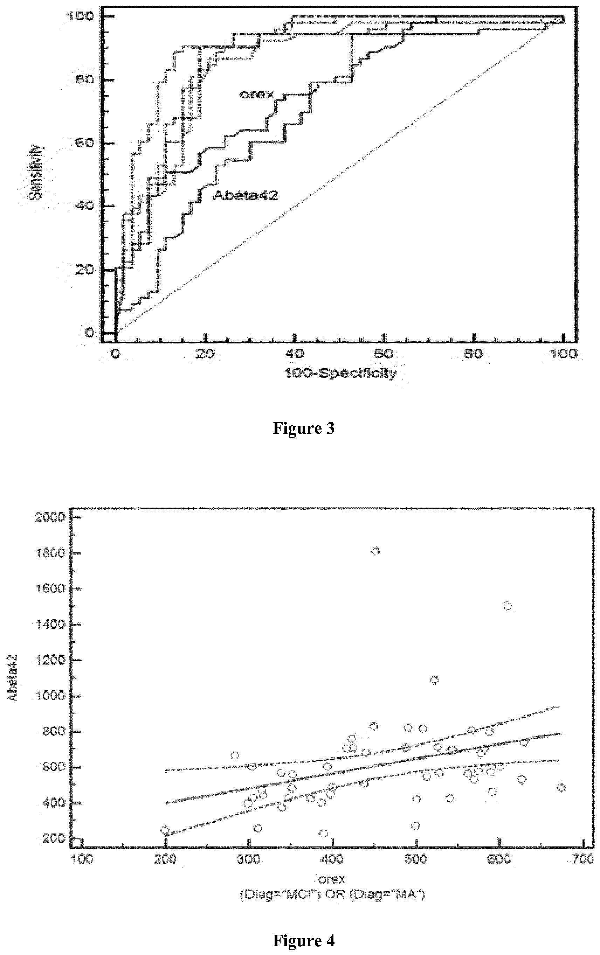

**Figure 3****:** Representation of the ROC curves of the individual biomarkers and of the optimal combination of biomarkers using logistic regression obtained with or without orexin-A/Hypocretin-1. The specificity is represented on the abscissa axis, the sensitivity is represented on the ordinate axis. The representation of the biomarkers or combination thereof, is as follows, with "Abeta42" and "Orex" designating respectively on the figure the corresponding continuous line:

| |
|---|
| - Abéta42 |
| ----------- tau |
| .............. ptau |
| ............... Abéta42*-0,0018682+ptau*0,041857+orex*0,0095223- |
| ................ Abéta42*-0,0018477+ptau*0,042620-1,5415 |
| - orex |

**Figure 4****:** Representation of the correlation between orexin-A concentration in ng/L (abscissa axis) and Aβ42 concentration in ng/L (ordinate axis) for the patients of the MCI and AD clinical group. Aβ42 and orexin-A are correlated in the AD/MCI diagnosis group only.

### EXAMPLES

### Example 1: Detection of orexin-A and other biomarkers in neurological diseases.

### 1. Subjects

One hundred and six unrelated patients (60 males and 46 females, median age 70.5 years, with a range between 38.0 and 92.0 years old) were included. All patients had an extensive examination including physical, neurologic and neuropsychological evaluations, laboratory tests and brain imaging. Blinded with respect to CSF test results, diagnoses of patients were set by consensus in a multidisciplinary team of neurologists, geriatricians and neuropsychologists. 91 patients with cognitive complaints were selected, including 37 subjects with AD, 16 with amnesic mild cognitive impairment (MCI) and 38 with others dementia (DEM). Patients with AD fit clinical diagnosis criteria as defined by the NINCDS-ADRDA. Patients with MCI initially meet the usual criteria established by Petersen, all subjects with a 2-to-7-year follow-up revealing progressive cognitive decline to AD. The DEM group included fronto-temporal lobar degeneration (FTLD) according to the consensus criteria for FTLD, dementia with Lewy body (DLB) according to the McKeith criteria for DLB and corticobasal degeneration with the Boeve *et al*., criteria. Dementia severity was assessed using the Mini-Mental State Examination (MMSE). Patients with typical narcolepsy-narcolepsy (NC) were included (n=15, 9 males and 6 females, median age 65.6 years old with a range between 54.0 and 86.4 years old). None of patients with NC had cognitive problems.

### 2. CSF samples and assays

CSF was obtained by lumbar puncture after a median duration of one month (interquartile range [IQR] 1-3 months) after diagnosis of cognitive alteration, except for NC patients, with more than 30 years of delay between symptoms onset and lumbar puncture in this particular old population sample. CSF was collected in polypropylene tubes with standardized conditions preferably between 11:00 and 13:00 to minimize the influence of diurnal variation of CSF Aβ₄₂ levels. Each CSF sample was transferred within 4 hours after collection and was centrifuged at 1,000 g for 10 minutes at 4°C. A small amount of CSF was used for routine analyses, including total cell count, bacteriological examination, total protein and glucose levels. CSF was aliquoted in polypropylene tubes of 1.5 mL and stored at -80°C until further analysis. CSF Aβ₄₂, total Tau and Phospho-Tau-181 (P-Tau) were measured using standardized commercially available INNOTEST® sandwich ELISA according to manufacturer's procedures (Innogenetics Ghent Belgium). All the three biomarkers were simultaneously analyzed in every CSF sample. From these measurements, Innotest® Amyloid Tau Index (IATI) was also calculated for each patient. We used the validated cut-offs for these biomarkers for clinical use in discriminating AD from normal aging and other neurologic disorders (Hulstaert *et al.*, 1999). The samples were thawed one more time for CSF orexin-A measurements. CSF orexin-A (orexin-A) was determined in all subjects in duplicate using ¹²⁵I radioimmunoassay kits from Phoenix Peptide, Inc, according to the manufacturer's prescriptions. The detection limit was 10 pg/mL and intra-assay variability was < 10%. CSF orexin-A levels <110 pg/mL were considered as low, intermediate between 110 and 200, and normal > 200. All values were back-referenced to Stanford reference samples (HHMI Stanford University Center for Narcolepsy, Palo Alto CA). The biological teams involved in CSF analysis were unaware of the clinical diagnosis.

### 3. Statistical analysis

The sample is described using percentages for categorical variables and median and range for quantitative variables (age, CSF measurements) as their distributions were tested with the Shapiro-Wilk statistic and were skewed. Age, sex, MMSE Score, and CSF measurements were compared between the four diagnosis groups (AD, MCI, DEM and NC) using Chi square for categorical variables and Kruskall-Wallis test for continuous variables. The comparison of two groups were done using Chi square (categorical variable) and Mann-Whitney Test (continuous variable). When comparisons were statistically significant, two-by-two comparisons were carried out, using the Bonferroni correction. Spearman's rank order correlations were applied to determine associations between two continuous variables. In order to determine which CSF measurements were independently associated with AD/MCI, the measurements were entered together in the same logistic regression model with potential confounders (age). Associations were thus quantified with odds ratios (OR) and their 95% confidence intervals (CI). Significance level was set at p < 0.05. Statistical analyses were performed using SAS, version 9.2 (SAS Institute, Cary, NC, USA).

### 4. Results

### 4.1. Biological comparisons between AD, MCI, DEM and NC

Table 2 summarizes the socio-demographic, clinical and biological variables of patients with different etiologies of cognitive impairment and patients with narcolepsy-cataplexy.

**Table 2**

| | AD N=37 | | MCI N=16 | | DEM N=38 | | NC N=15 | | p-value | p-value⁽¹⁾ |
|---|---|---|---|---|---|---|---|---|---|---|
| Age (in years) | 72.30 [49.80-89.49] | | 73.20 [57.39-84.39] | | 66.84 [32.07-85.15] | | 65.59 [54.04-86.38] | | 0.03 | |
| Gender | | | | | | | | | | |
| Male | 17 | 45 | 10 | 62 | 24 | 63 | 9 | 60 | 0.44 | |
| Female | 20 | 54 | 6 | 37 | 14 | 36 | 6 | 40 | 4C | |
| MMSE | 21.00 [2.00-25.00] | | 26.00 [26.00-30.00] | | 20.50 [9.00-30.00] | | 30.00 [30.00-30.00] | | <0.0001 | |
| MMSE | | | | | | | | | | |
| <26 | 35 | 100 | 0 | | 29 | 80 | 0 | 0 | <0.0001 | |
| ≥26 | 0 | 0 | 14 | 100 | 7 | 19 | 15 | 100 | 100 | |
| CSF biomarker measurement | | | | | | | | | | |
| Aβ1-42 (pg/mL) | 563.00 [232.00-1811.00] | | 593.00 [273.00-1505.00] | | 694.00 [306.00-1645.00] | | 947.00 [566.00-1332.00] | | 0.0005 | 0.02 |
| Aβ1-42 (pg/mL) | | | | | | | | | | |
| ≤500 | 14 | 37 | 5 | 31 | 8 | 21 | 0 | 0 | NA | NA |
| >500 | 23 | 62 | 11 | 68 | 30 | 78 | 15 | 100 | 100 | |
| Tau (pg/mL) | 579.00 [283.00-2165.00] | | 706.50 [343.00-1200.00] | | 264.50 [104.00-1200.00] | | 215.00 [69.00-633.00] | | <0.0001 | <0.0001 |
| Tau (pg/mL) | | | | | | | | | | |
| ≥ cut-off ⁽²⁾ | 26 | 70 | 11 | 68 | 12 | 31 | 1 | 6 | <0.0001 | - |
| <cut-off | 11 | 29 | 5 | 31 | 26 | 68 | 14 | 93 | | |
| P-Tau (pg/mL) | 79.00 [20.00-215.00] | | 106.50 [46.00-215.00] | | 41.50 [8.00-156.00] | | 41.00 [19.00-92.00] | | <0.0001 | <0.0001 |
| P-Tau (pg/mL) | | | | | | | | | | |
| ≥60 | 32 | 86 | 14 | 87 | 7 | 18 | 4 | 26 | <0.0001 | <0.0001 |
| <60 | 5 | 13 | 2 | 12 | 31 | 81 | 11 | 73 | | |
| IATI Index | 0.62 [0.00-2.53] | | 0.60 [0.29-2.33] | | 1.28 [0.34-3.55] | | 1.76 [1.05-2.28] | | <0.0001 | <0.0001 |
| IATI Index | | | | | | | | | | |
| <1 | 33 | 89 | 14 | 87 | 16 | 43 | 0 | 0 | <0.0001 | 0.0009 |
| ≥1 | 4 | 10 | 2 | 12 | 21 | 56 | 15 | 100 | 100 | |
| Hypocretin-1 (pg/mL) | 451.00 [199.00-672.50] | | 503.75 [299.00-628.50] | | 386.00 [273.00-574.00] | | 34.00 [10.00-108.00] | | 0.03 | 0.06 |
| Hypocretin- 1 (pg/mL) | | | | | | | | | | |
| <110 | 0 | 0 | 0 | 0 | 0 | 0 | 15 | 100 | NA | NA |
| 110-200 | 1 | 2 | 0 | 0 | 0 | 0 | 0 | 0 | 0 | |
| ≥200 | 36 | 97 | 16 | 100 | 38 | 100 | 0 | 0 | 0 | |

| | | | | | | | | | | |
|---|---|---|---|---|---|---|---|---|---|---|
| ⁽¹⁾ adjustment for age ⁽²⁾ 300 pg/mL (if age between 21 and 50 years old), 450 (if age between 51 and 70 years old) and 500 (if age>71 years old) NA : Not Applicable | | | | | | | | | | |

Table 3 summarizes the socio-demographic, clinical and biological variables between AD-MCI patients and patients with other etiology of dementia. CSF biomarker levels including Aβ₄₂, Tau, P-Tau, and IATI significantly differed between groups using both median and validated cut-offs (Figure 1).

**Table 3**

| | AD + MCI N=53 | | DEM N=38 | | p-value | p-value⁽¹⁾ |
|---|---|---|---|---|---|---|
| Age (in years) | 72.58 [49.80-89.49] | | 66.84 [32.07-85.15] | | 0.02 | |
| Gender | | | | | | |
| Male | 27 | 50.94 | 24 | 63.16 | 0.25 | |
| Female | 26 | 49.06 | 14 | 36.84 | | |
| MMSE | 23.00 [2.00-30.00] | | 20.50 [9.00-30.00] | | 0.09 | |
| MMSE | | | | | | |
| <26 | 35 | 71.43 | 29 | 80.56 | 0.34 | |
| ≥26 | 14 | 28.57 | 7 | 19.44 | | |
| CSF biomarker | | | | | | |
| Aβ1-42 (pg/mL) | 568.00 [232.00- | | 694.00 [306.00- | | 0.01 | 0.03 |
| Aβ1-42 (pg/mL) | | | | | | |
| ≤500 | 19 | 35.85 | 8 | 21.05 | 0.13 | 0.19 |
| >500 | 34 | 64.15 | 30 | 78.95 | | |
| Tau (pg/mL) | 588.00 [283.00- | | 264.50 [104.00- | | <0.0001 | <0.0001 |
| Tau (pg/mL) | | | | | | |
| ≥ cut-off ⁽²⁾ | 37 | 69.81 | 12 | 31.58 | <0.0001 | |
| <cut-off | 16 | 30.19 | 26 | 68.42 | | |
| P-Tau (pg/mL) | 82.00 [20.00-215.00] | | 41.50 [8.00-156.00] | | <0.0001 | <0.0001 |
| P-Tau (pg/mL) | | | | | | |
| ≥60 | 46 | 86.79 | 7 | 18.42 | <0.0001 | <0.0001 |
| <60 | 7 | 13.21 | 31 | 81.58 | | |
| IATI index | 0.60 [0.00-2.53] | | 1.28 [0.34-3.55] | | <0.0001 | <0.0001 |
| IATI Index | | | | | | |
| <1 | 47 | 88.68 | 16 | 43.24 | <0.0001 | <0.0001 |
| ≥1 | 6 | 11.32 | 21 | 56.76 | | |
| Hypocretin-1 (pg/mL) | 486.50 [199.00- | | 386.00 [273.00- | | 0.004 | 0.005 |
| Hypocretin-1 (pg/mL) | | | | | | |
| 110-200 | 1 | 1.89 | 0 | 0.00 | 0.58 | NA |
| ≥200 | 52 | 98.11 | 38 | 100.00 | | |

| | | | | | | |
|---|---|---|---|---|---|---|
| *⁽¹⁾ adjustment for age: 300 pg*/*mL (if age between 21 and 50 years old), 450 (if age between 51 and 70 years old) and 500 (if age>71 years old)* | | | | | | |

More precisely CSF Aβ₄₂ levels differed between AD and NC (p<0.0006), and MCI and NC groups (p=0.013). Significant between-group differences were also found for CSF Tau, P-Tau and IATI levels in AD vs DEM, AD vs NC, MCI vs DEM, and MCI vs NC populations (p<0.006 for all the comparisons after the Bonferroni correction). None of patients with NC achieved pathological cut-offs of Aβ₄₂ or IATI, with respectively one and four patients with NC above Tau and P-Tau cut-offs (Table 2). As expected CSF orexin-A levels differed between NC and all other diagnosis groups (all p<0.0001) but also between MCI and DEM (p=0.04) (Figure 2). No other two-by-two comparisons revealed significant differences. One patient with diagnosis of MCI had intermediate CSF orexin-A levels but none of patients with cognitive impairment had low levels.

### 4.2. Biological comparisons between AD+MCI and DEM

All CSF biomarker levels differed between AD/MCI and DEM groups with lower levels of Aβ₄₂ and IATI and higher levels of Tau and P-Tau in AD/MCI condition. CSF orexin-A levels also differed between AD/MCI and DEM groups with higher levels in the former diagnosis.

### 4.3. Contribution of each CSF biomarker for diagnosis

Two multivariate logistic regression models were carried out to identify relative contribution of CSF biomarker measurements to AD/MCI (see Table 4). The first model included orexin-A, Tau, Aβ₄₂ and age (model 1) and the second included orexin-A, P-Tau, Aβ₄₂ and age (model 2).

**Table 4**

| Model 1 | | Model 2 | |
|---|---|---|---|
| *Variables* | *OR [95%CI]* | *Variables* | *OR [95%CI]* |
| Hypocretin-1 (pg/mL) OR for 110-unit increase | 2.70 [1.37- 5.32] | Hypocretin-1 (pg/mL) OR for 110-unit increase | 2.66 [1.35-5.23] |
| Tau (pg/mL) OR for 300-unit increase | 4.24 [2.03-8.84] | p-Tau (pg/mL) OR for 60-unit increase | 12.49 [3.61-43.23] |
| Aβ1-42 (pg/mL) OR for 500-unit increase | 0.52 [0.20-1.33] | Aβ1-42 (pg/mL) OR for 500-unit increase | 0.44 [0.17-1.14] |
| Age OR for 10-year increase | 2.15 [1.05-4.40] | Age OR for 10-yearsincrease | 1.94 [0.96-3.92] |

Table 5 summarizes the area under curve (AUC) of the ROC curves of the individual biomarkers and of the combination of two biomarkers.

**Table 5**

| AUC ROC | Abeta42 | Tau | Ptau | Hypocretin-1 |
|---|---|---|---|---|
| Abeta42 | 0.707 | 0.875 | 0.873 | 0.841 |
| Tau | | 0.882 | 0.876 | 0.896 |
| Ptau | | | 0.857 | 0.906 |
| Hypocretin-1 | | | | 0.769 |

Table 6 presents a multivariate logistic regression model of CSF biomarker measurements associated with AD/MCI. SE is defined according to Delong *et al.* (1988).

**Table 6**

| | AUC | SE^{a} | 95% CI^{b} |
|---|---|---|---|
| Abeta42 | 0.707 | 0.0511 | 0.611 to 0.791 |
| tau | 0.882 | 0.0342 | 0.804 to 0.936 |
| ptau | 0.857 | 0.0373 | 0.776 to 0.918 |
| Logistic Regression with OREXIN Abéta42^{∗}-0,0018682+ptau^{∗}0,041857+orexv^{∗}0,0095223- | 0.917 | 0.0280 | 0.847 to 0.962 |
| Logistic Regression without OREXIN Abéta42^{∗}-0,0018477+ptau^{∗}0,042620-1,5415 | 0.874 | 0.0358 | 0.795 to 0.930 |
| Orex | 0.769 | 0.0447 | 0.678 to 0.846 |

After adjustment for these variables, orexin-A remained significantly and independently associated with AD/MCI with an OR of 2.70 (95%CI=[1.37- 5.32]) for the model 1 and OR=2.66 (95%CI=[1.35-5.23]) for the model 2. The contribution of CSF orexin-A is greater than that of the Aβ₄₂ (OR of 0.52 (95%CI=[0.20- 1.33]) for the model 1 and OR=0.44 (95%CI=[0.17-1.14]) for the model 2 (Figure 3).

### 4.4. Relationships between each biomarker in AD+MCI, DEM and NC groups of patients

We further analyzed the relationships between biomarkers in the entire group of 91 patients with cognitive complaints and revealed negative correlations between Aβ₄₂ and Tau (r=-0.3, p=0.0046), P-Tau (r=-0.22, p=0.04) and as expected between Tau and P-Tau, but without any with orexin-A levels. However in the group AD/MCI (n=53), we found a positive correlation between orexin-A levels and Aβ₄₂ (r=0.43, p=0.0013), and IATI index (r=0.42, p=0.0017), findings mostly driven by patients with AD (r=0.45, p=0.0052). No correlation were found between orexin-A and other biomarkers in DEM. Unexpectedly, we found positive correlations between CSF Aβ₄₂ and Tau (r=0.69, p=0.0042), and P-tau (r=0.70, p=0.0037) in patients with NC, without any between orexin-A and other biomarkers (Figure 4).

### 5. Conclusion

The levels of CSF Aβ₄₂, Tau, P-Tau and orexin-A (Hypocretin-1) are reported among patients with different etiologies of cognitive impairments together with their results in hypocretin-deficient narcoleptic patients. Higher CSF orexin-A levels are reported in AD/MCI condition and the results confirm the lower CSF Aβ₄₂ and higher Tau and P-Tau levels in AD and MCI conditions compared to patients with other etiologies of dementia. Results show that orexin-A is significantly and independently associated with AD/MCI with an OR of 2.70 after full-adjustment. A positive correlation was found between Aβ₄₂ and orexin-A levels in the AD/MCI group only, without any association with clinical sleep disturbances. This study reveals that the relative contribution of orexin-A in diagnosing AD/MCI compared to other dementia is higher than using Aβ₄₂. The major impact of this result is to underline that orexin-A is significantly and independently associated with AD/MCI with an OR of 2.70 after full-adjustment. Although only one patient with MCI had intermediate CSF orexin-A level, our study revealed that the relative contribution of orexin-A in diagnosing AD/MCI compared to other dementia was higher than using Aβ₄₂. Another striking finding of the present study is the positive correlation between orexin-A and Aβ₄₂ levels in AD/MCI condition but neither in other dementia nor in NC. As patients with MCI included were regularly seen in the CMRR with follow-up revealing progressive cognitive decline to AD, we were able to precise that brain β-amyloid and orexin-A interactions particularly occurred in advanced stage of AD process (mostly in patients with AD instead of MCI).

Daytime wake and nighttime sleep fragmentations were frequently reported in AD with unclear biologic basis. As hypocretin is a major wake-related neurotransmitter, one study focused on its impact on the consolidation of wakefulness in AD (Friedman *et al*., 2007). Using wrist actigraphy recording, the results mainly revealed an increased wake fragmentation in patients with lower CSF orexin-A levels. We took advantage of CSF samples from old patients with NC characterized by daytime sleepiness, fragmented nighttime sleep, cataplexy and particularly hypocretin deficiency. As expected CSF Aβ₄₂, Tau, P-Tau and IATI levels significantly differed between AD, MCI and NC groups. Patients with NC with low CSF orexin-A levels did not present low Aβ₄₂ levels, thus positive correlation reported between CSF Aβ42 and orexin-A levels in AD/MCI condition seems specific to AD pathogeny.

The diagnosis interest of hypocretin is highlighted by the fact that its CSF concentrations were significantly different not only between DEM and AD patients, but also between DEM and MCI patients that eventually converted into AD. The latter observation indicates that hypocretin could be used for the early detection of AD. There is a rationale for this, since amyloid pathology is believed to be present very early in the development of the disease, and since Aβ42 and hypocretin were correlated in AD.

### Example 2: Determination of the concentration of orexin-A in biological samples

### 1. CSF samples

CSF is collected by lumbar puncture in polypropylene tubes with standardized conditions, at standardized times, and preferably between 11:00 and 13:00 to minimize the influence of diurnal variation of the biomarkers levels. Each CSF sample is transferred within 4 hours after collection and centrifuged at 1,000 g for 10 minutes at 4°C. A small amount of CSF may be used for routine analyses (total cell count, bacteriological exam, total protein and glucose levels). CSF is then aliquoted in polypropylene tubes of 1.5 mL and stored at -80°C until further analysis.

### 2. Assays

CSF Aβ₄₂, total Tau and Phospho-Tau-181 (P-Tau) are simultaneously measured using standardized commercially available INNOTEST® sandwich ELISA according to manufacturer's procedures (Innogenetics Ghent Belgium). From these measurements, Innotest® Amyloid Tau Index (IATI= Abeta/(240+1,18^{∗}Tau) is calculated for each patient, using the formula IATI=Aβ42/(240+1.18×tau) as described (Hulstaert, et al., 1999). The validated cut-offs for these biomarkers for clinical use in discriminating AD from normal aging and other neurologic disorders can be used (Hulstaert *et al.*, 1999). CSF samples are thawed one more time for orexin-A measurements. CSF orexin-A is determined in all subjects in duplicate using ¹²⁵I radioimmunoassay kits from Phoenix Peptide, Inc, according to the manufacturer's prescriptions. The detection limit is 10 pg/mL and intra-assay variability is < 10%.

### 3. Analysis

CSF orexin-A levels <110 pg/mL are considered as low, intermediate between 110 and 200, and normal > 200. An index of the combined concentration values chosen among the following is calculated:
- Concentration of Orexin-A, Tau and Aβ₄₂,
- Concentration of Orexin-A, Phospho-Tau and Aβ₄₂
- Concentration of Orexin-A and Tau,
- Concentration of Orexin-A and Phospho-Tau

### BIBLIOGRAPHIC REFERENCES

Duyckaerts C, et al., Acta Neuropathol. Jul;118(1):5-36 (2009).
Braak and Braak, Acta Neurol Scand Suppl.; 165:3-12 (1996)
Marksteiner J. et al., Pharmacology. 2008;82(3):214-20.
Blennow K, Zetterberg H. J Alzheimers Dis;18:413-417 (2009)
Mattsson N, et al., Jama;302:385-393 (2009).
Gabelle A et al., J Alzheimers Dis. Jan 1;26(3):553-63 (2011)
Dauvilliers et al., J Neurol Neurosurg Psychiatry. Dec;74(12): 1667-73 (2003)
Baumann CR et al., Eur Neurol 52, 73-76 (2004)
Wennström M et al., J Alzheimers Dis.;29(1):125-32 (2012)
Fronczek R et al., Neurobiol Aging, Aug;33(8):1642-50 (2012)
Kang JE et al., Science 326, 1005-1007 (2009)
Slats D et al., Ageing Res Rev., Jan;12(1):188-200 (2013)
Dauvilliers Y et al., Lancet. Feb 10;369 (9560):499-511 (2007)
Maniatis T. *et al.*, Cold Spring Harbor Laboratory, Cold Spring Harbor, N.Y, Edition (1999)
DeLong ER, et al., Biometrics 44: 837-845 (1988).
Hulstaert, F. et al., et al., Neurology 52, 1555-1562 (1999).
Schmidt F.M., et al., PlOS ONE, 2013;8(5), e63136:1-6 9[
Yasui K., et al., J. Neurol. Sci., 2006;250(1-2):120-123
Gabelle A., et al., Revue Neurologique, 2009;165(3):213-222
Gabelle A., et al., J. Alzheimer Dis., 2013;34(1):297-305

## Claims

1. Method for the in vitro diagnosis of a neurological disorder, said method comprising the steps of:
a) determining the level or the concentration of a compound chosen in the group consisting of orexin-A, a specific fragment thereof which can be specifically detected by a specific ligand of orexin-A, an isoform thereof, a precursor thereof, a nucleic acid encoding the same and a combination thereof, in a test sample from a subject,
b) comparing the level or the concentration of said compound in said test sample with the level or the concentration of the same compound in a reference sample,
c) determining from the comparison of step b) if said subject is affected by said neurological disorder, wherein a higher orexin-A level is indicative of said neurological disorder,
wherein the neurological disorder is a dementia chosen in the group consisting of: Alzheimer's disease (AD) and mild cognitive impairment (MCI), and the reference sample is collected from subjects affected by a dementia excepting AD and MCI and wherein said test sample and said reference sample are biological samples chosen in the group consisting of: Cerebrospinal Fluid (CSF), serum, plasma, whole blood and a whole blood extract.

2. Method according to claim 1 , comprising the steps of:
a) determining in a test sample from a subject the concentration of a compound chosen in the group consisting of: orexin-A, a specific fragment thereof, an isoform thereof, a precursor thereof, a nucleic acid encoding the same and a combination thereof, and of at least one biological marker of AD,
b) comparing the concentration of said compound in said test sample with the concentration of the same compound in a reference sample, and comparing the concentration of said at least one biological marker of AD in said test sample with the concentration of said at least one biological marker of AD in a reference sample,
c) determining from the comparisons of step b) if said subject is affected by said neurological disorder.

3. Method according to claim 2, wherein said at least one biological marker of AD is chosen in the group consisting of: Tau, Phospho-Tau, Aβ₄₂, a specific fragment thereof, an isoform thereof, a precursor thereof, a nucleic acid encoding the same and a combination thereof.

4. Method according to any one of claims 1 to 3, comprising the determination of the concentration of orexin-A, a specific fragment thereof, an isoform thereof, a precursor thereof, a nucleic acid encoding the same or a combination thereof, and the determination of the concentration of Tau, a specific fragment thereof, an isoform thereof, a precursor thereof, a nucleic acid encoding the same or a combination thereof.

5. Method according to any one of claims 1 to 3, comprising the determination of the concentration of orexin-A, a specific fragment thereof, an isoform thereof, a precursor thereof, a nucleic acid encoding the same or a combination thereof, and the determination of the concentration of Phospho-Tau, a specific fragment thereof, an isoform thereof, a precursor thereof, a nucleic acid encoding the same or a combination thereof.

6. Method according to any one of claims 1 to 3, comprising the determination of the concentration of orexin-A, a specific fragment thereof, an isoform thereof, a precursor thereof, a nucleic acid encoding the same and a combination thereof, and the determination of the concentration of Aβ₄₂, a specific fragment thereof, an isoform thereof, a precursor thereof, a nucleic acid encoding the same or a combination thereof.

7. Method according to any one of claims 1 to 6, wherein said test sample and said reference sample are Cerebrospinal Fluid (CSF) samples.

8. Method according to any one of claims 1 to 7, wherein the concentration of orexin-A, a specific fragment thereof or a combination thereof, is determined by a method chosen in the group consisting of: a method based on immuno-detection, a method based on mass spectrometry, a method based on western blot, a method based on liquid chromatography, a method based on flow cytometry.

9. Use of orexin-A, a specific fragment thereof which can be specifically detected by a specific ligand of orexin-A, an isoform thereof, a precursor thereof, a nucleic acid encoding the same or a combination thereof as a biomarker in the in vitro diagnosis method of claim 1 of a neurological disorder chosen in the group consisting of AD and MCI.

10. Use in the method of claim 1 of a kit for the in vitro diagnosis of a neurological disorder chosen in the group consisting of AD and MCI, comprising a reagent for the specific detection of a compound chosen in the group consisting of orexin-A, a specific fragment thereof which can be specifically detected by a specific ligand of orexin-A, an isoform thereof, a precursor thereof, a nucleic acid encoding the same and a combination thereof, and at least one compound chosen in the group consisting of buffers, positive controls and instructions for use.

11. Use of a kit according to claim 10, comprising at least one reagent for the detection of at least one biological marker of AD chosen in the group consisting of: Tau, Phospho-Tau, Aβ₄₂, a specific fragment thereof, an isoform thereof, a precursor thereof, a nucleic acid encoding the same and a combination thereof.

12. Use of a kit according to claim 10 or 11 wherein said reagent specific for orexin-A, a specific fragment thereof, an isoform thereof, a precursor thereof, a nucleic acid encoding the same or a combination thereof is chosen in the group consisting of a ligand of orexin-A and a ligand of a nucleic acid encoding the same.

## Patentansprüche

1. Verfahren zur In-vitro-Diagnose einer neurologischen Erkrankung oder Störung, wobei das Verfahren folgende Schritte umfasst:
a) Bestimmen des Gehalts oder der Konzentration einer Verbindung, ausgewählt aus der Gruppe bestehend aus Orexin-A, einem spezifischen Fragment davon, das durch einen spezifischen Liganden von Orexin-A spezifisch nachgewiesen werden kann, einer Isoform davon, einem Vorläufer davon, einer dafür kodierenden Nukleinsäure und einer Kombination davon, in einer Untersuchungsprobe von einer Testperson,
b) Vergleichen des Gehalts oder der Konzentration der Verbindung in der Untersuchungsprobe mit dem Gehalt oder der Konzentration der gleichen Verbindung in einer Vergleichsprobe,
c) Bestimmen, anhand des Vergleichs aus Schritt b), ob die Testperson von der neurologischen Erkrankung oder Störung betroffen ist, wobei ein höherer Orexin-A-Gehalt auf die neurologische Erkrankung oder Störung hinweist,
wobei die neurologische Erkrankung oder Störung eine Demenz ist, ausgewählt aus der Gruppe bestehend aus: Alzheimer-Krankheit (AD) und leichter kognitiver Störung (MCI), und die Vergleichsprobe von Testpersonen entnommen ist, die von einer Demenz, mit Ausnahme von Alzheimer-Krankheit und MCI, betroffen sind, und wobei die Untersuchungsprobe und die Vergleichsprobe biologische Proben sind, ausgewählt aus der Gruppe bestehend aus: Liquor (Liquor cerebrospinalis, LCS), Serum, Plasma, Vollblut und Vollblutextrakt.

2. Verfahren nach Anspruch 1, das folgende Schritte umfasst:
a) Bestimmen, in einer Untersuchungsprobe von einer Testperson, der Konzentration einer Verbindung, ausgewählt aus der Gruppe bestehend aus: Orexin-A, einem spezifischen Fragment davon, einer Isoform davon, einem Vorläufer davon, einer dafür kodierenden Nukleinsäure und einer Kombination davon, sowie mindestens eines Biomarkers der Alzheimer-Krankheit,
b) Vergleichen der Konzentration der Verbindung in der Untersuchungsprobe mit der Konzentration der gleichen Verbindung in einer Vergleichsprobe, und Vergleichen der Konzentration des mindestens einen Biomarkers der Alzheimer-Krankheit in der Untersuchungsprobe mit der Konzentration des mindestens einen Biomarkers der Alzheimer-Krankheit in einer Vergleichsprobe,
c) Bestimmen, anhand der Vergleiche aus Schritt b), ob die Testperson von der neurologischen Erkrankung oder Störung betroffen ist.

3. Verfahren nach Anspruch 2, wobei der mindestens eine Biomarker der Alzheimer-Krankheit ausgewählt ist aus der Gruppe bestehend aus: Tau, Phospho-Tau, Aß₄₂, einem spezifischen Fragment davon, einer Isoform davon, einem Vorläufer davon, einer dafür kodierenden Nukleinsäure und einer Kombination davon.

4. Verfahren nach einem der Ansprüche 1 bis 3, umfassend das Bestimmen der Konzentration von Orexin-A, einem spezifischen Fragment davon, einer Isoform davon, einem Vorläufer davon, einer dafür kodierenden Nukleinsäure oder einer Kombination davon, und das Bestimmen der Konzentration von Tau, einem spezifischen Fragment davon, einer Isoform davon, einem Vorläufer davon, einer dafür kodierenden Nukleinsäure oder einer Kombination davon.

5. Verfahren nach einem der Ansprüche 1 bis 3, umfassend das Bestimmen der Konzentration von Orexin-A, einem spezifischen Fragment davon, einer Isoform davon, einem Vorläufer davon, einer dafür kodierenden Nukleinsäure oder einer Kombination davon, und das Bestimmen der Konzentration von Phospho-Tau, einem spezifischen Fragment davon, einer Isoform davon, einem Vorläufer davon, einer dafür kodierenden Nukleinsäure oder einer Kombination davon.

6. Verfahren nach einem der Ansprüche 1 bis 3, umfassend das Bestimmen der Konzentration von Orexin-A, einem spezifischen Fragment davon, einer Isoform davon, einem Vorläufer davon, einer dafür kodierenden Nukleinsäure und einer Kombination davon, und das Bestimmen der Konzentration von Aß₄₂, einem spezifischen Fragment davon, einer Isoform davon, einem Vorläufer davon, einer dafür kodierenden Nukleinsäure oder einer Kombination davon.

7. Verfahren nach einem der Ansprüche 1 bis 6, wobei die Untersuchungsprobe und die Vergleichsprobe Liquorproben sind.

8. Verfahren nach einem der Ansprüche 1 bis 7, wobei die Konzentration von Orexin-A, einem spezifischen Fragment davon oder einer Kombination davon bestimmt wird durch ein Verfahren, ausgewählt aus der Gruppe bestehend aus: einem auf Immundetektion beruhenden Verfahren, einem auf Massenspektrometrie beruhenden Verfahren, einem auf Western Blot beruhenden Verfahren, einem auf Flüssigchromatographie beruhenden Verfahren, einem auf Durchflusszytometrie beruhenden Verfahren.

9. Verwendung von Orexin-A, einem spezifischen Fragment davon, das durch einen spezifischen Liganden von Orexin-A spezifisch nachgewiesen werden kann, einer Isoform davon, einem Vorläufer davon, einer dafür kodierenden Nukleinsäure oder einer Kombination davon als ein Biomarker im In-vitro-Diagnose-Verfahren nach Anspruch 1 für eine neurologische Erkrankung oder Störung, ausgewählt aus der Gruppe bestehend aus Alzheimer-Krankheit und MCI.

10. Verwendung, im Verfahren nach Anspruch 1, eines Kits für die In-vitro-Diagnose einer neurologischen Erkrankung oder Störung, ausgewählt aus der Gruppe bestehend aus Alzheimer-Krankheit und MCI, umfassend ein Reagens für den spezifischen Nachweis einer Verbindung, ausgewählt aus der Gruppe bestehend aus Orexin-A, einem spezifischen Fragment davon, das durch einen spezifischen Liganden von Orexin-A spezifisch nachgewiesen werden kann, einer Isoform davon, einem Vorläufer davon, einer dafür kodierenden Nukleinsäure und einer Kombination davon, sowie mindestens eine Verbindung, ausgewählt aus der Gruppe bestehend aus Puffern, Positivkontrollen und Gebrauchsanleitungen.

11. Verwendung eines Kits nach Anspruch 10, umfassend mindestens ein Reagens für den Nachweis von mindestens einem Biomarker der Alzheimer-Krankheit, ausgewählt aus der Gruppe bestehend aus: Tau, Phospho-Tau, Aß₄₂, einem spezifischen Fragment davon, einer Isoform davon, einem Vorläufer davon, einer dafür kodierenden Nukleinsäure und einer Kombination davon.

12. Verwendung eines Kits nach Anspruch 10 oder 11, wobei das für Orexin-A, ein spezifisches Fragment davon, eine Isoform davon, einen Vorläufer davon, eine dafür kodierende Nukleinsäure oder eine Kombination davon spezifische Reagens ausgewählt ist aus der Gruppe bestehend aus einem Liganden von Orexin-A und einem Liganden einer dafür kodierenden Nukleinsäure.

## Revendications

1. Procédé pour le diagnostic in vitro d'un trouble neurologique, ledit procédé comprenant les étapes suivantes :
a) détermination du niveau ou de la concentration d'un composé choisi dans le groupe constitué par l'orexine A, un fragment spécifique de celle-ci qui peut être spécifiquement détecté par un ligand spécifique d'orexine A, une isoforme de celle-ci, un précurseur de celle-ci, un acide nucléique la codant, et une de leurs combinaisons, dans un échantillon de test provenant d'un sujet,
b) comparaison du niveau ou de la concentration dudit composé dans ledit échantillon de test avec le niveau ou la concentration du même composé dans un échantillon de référence,
c) détermination, à partir de la comparaison de l'étape b), que le sujet est ou non affecté par ledit trouble neurologique, dans laquelle un niveau d'orexine A plus élevé est indicatif dudit trouble neurologique,
dans lequel le trouble neurologique est une démence choisie dans le groupe constitué par la maladie d'Alzheimer (AD) et un trouble cognitif léger (MCI), et l'échantillon de référence est collecté auprès de sujets affectés d'une démence à l'exception de l'AD et d'un MCI, et dans lequel ledit échantillon de test et ledit échantillon de référence sont des échantillons biologiques choisis dans le groupe constitué par : le liquide céphalo-rachidien (CSF), le sérum, le plasma, le sang entier et un extrait de sang entier.

2. Procédé selon la revendication 1, comprenant les étapes suivantes :
a) détermination, dans un échantillon de test provenant d'un sujet, de la concentration d'un composé choisi dans le groupe constitué par : l'orexine A, un fragment spécifique de celle-ci, une isoforme de celle-ci, un précurseur de celle-ci, un acide nucléique la codant, et une de leurs combinaisons, et d'au moins un marqueur biologique d'AD,
b) comparaison de la concentration dudit composé dans ledit échantillon de test avec la concentration du même composé dans un échantillon de référence, et comparaison de la concentration dudit au moins un marqueur biologique d'AD dans ledit échantillon de test avec la concentration dudit au moins un marqueur biologique d'AD dans un échantillon de référence,
c) détermination, à partir des comparaisons de l'étape b), que le sujet est ou non affecté par ledit trouble neurologique.

3. Procédé selon la revendication 2, dans lequel ledit au moins un marqueur biologique d'AD est choisi dans le groupe constitué par : Tau, Phospho-Tau, Aβ42, un fragment spécifique de celui-ci, une isoforme de celui-ci, un précurseur de celui-ci, un acide nucléique le codant, et une de leurs combinaisons.

4. Procédé selon l'une quelconque des revendications 1 à 3, comprenant la détermination de la concentration d'orexine-A, d'un fragment spécifique de celle-ci, d'une isoforme de celle-ci, d'un précurseur de celle-ci, d'un acide nucléique la codant, ou d'une de leurs combinaisons, et la détermination de la concentration de Tau, d'un fragment spécifique de celle-ci, d'une isoforme de celle-ci, d'un précurseur de celle-ci, d'un acide nucléique la codant, ou d'une de leurs combinaisons.

5. Procédé selon l'une quelconque des revendications 1 à 3, comprenant la détermination de la concentration d'orexine-A, d'un fragment spécifique de celle-ci, d'une isoforme de celle-ci, d'un précurseur de celle-ci, d'un acide nucléique la codant, ou d'une de leurs combinaisons, et la détermination de la concentration de Phospho-Tau, d'un fragment spécifique de celle-ci, d'une isoforme de celle-ci, d'un précurseur de celle-ci, d'un acide nucléique la codant, ou d'une de leurs combinaisons.

6. Procédé selon l'une quelconque des revendications 1 à 3, comprenant la détermination de la concentration d'orexine-A, d'un fragment spécifique de celle-ci, d'une isoforme de celle-ci, d'un précurseur de celle-ci, d'un acide nucléique la codant, et d'une de leurs combinaisons, et la détermination de la concentration d'Aβ₄₂, d'un fragment spécifique de celui-ci, d'une isoforme de celui-ci, d'un précurseur de celui-ci, d'un acide nucléique le codant, ou d'une de leurs combinaisons.

7. Procédé selon l'une quelconque des revendications 1 à 6, dans lequel ledit échantillon de test et ledit échantillon de référence sont des échantillons de liquide céphalo-rachidien (CSF).

8. Procédé selon l'une quelconque des revendications 1 à 7, dans lequel la concentration d'orexine-A, d'un fragment spécifique de celle-ci, ou d'une de leurs combinaisons, est déterminée par un procédé choisi dans le groupe constitué par : un procédé basé sur une immunodétection, un procédé basé sur une spectrométrie de masse, un procédé basé sur un transfert Western, un procédé basé sur une chromatographie liquide, un procédé basé sur une cytométrie de flux.

9. Utilisation d'orexine-A, d'un fragment spécifique de celle-ci qui peut être spécifiquement détecté par un ligand spécifique d'orexine A, d'une isoforme de celle-ci, d'un précurseur de celle-ci, d'un acide nucléique la codant, ou d'une de leurs combinaisons, en tant que marqueur biologique dans le procédé de diagnostic in vitro de la revendication 1 d'un trouble neurologique choisi dans le groupe constitué par l'AD et un MCI.

10. Utilisation, dans le procédé de la revendication 1, d'un kit pour le diagnostic in vitro d'une maladie neurologique choisie dans le groupe constitué par l'AD et un MCI, comprenant un réactif pour la détection spécifique d'un composé choisi dans le groupe constitué par l'orexine-A, un fragment spécifique de celle-ci qui peut être spécifiquement détecté par un ligand spécifique d'orexine A, une isoforme de celle-ci, un précurseur de celle-ci, un acide nucléique la codant, et une de leurs combinaisons, et au moins un composé choisi dans le groupe constitué par les tampons, les témoins positifs et les instructions d'utilisation.

11. Utilisation d'un kit selon la revendication 10, comprenant au moins un réactif pour la détection d'au moins un marqueur biologique de l'AD choisi dans le groupe constitué par : Tau, Phospho-Tau, Aβ42, un fragment spécifique de celui-ci, une isoforme de celui-ci, un précurseur de celui-ci, un acide nucléique le codant, et une de leurs combinaisons.

12. Utilisation d'un kit selon la revendication 10 ou 11, dans laquelle ledit réactif spécifique de l'orexine A, d'un fragment spécifique de celle-ci, d'une isoforme de celle-ci, d'un précurseur de celle-ci, d'un acide nucléique la codant, ou d'une de leurs combinaisons, est choisi dans le groupe constitué par un ligand d'orexine A et un ligand d'un acide nucléique la codant.
